# EUROPEAN PATENT SPECIFICATION

(11) **EP 3 884 883 B1**
(45) Date of publication and mention of the grant of the patent: **12.06.2024**
(21) Application number: 21167456.9
(22) Date of filing: 23.01.2017
(51) Int. Cl.: A61B 17/22, A61M 1/00, A61M 3/02, A61B 90/00

(54) **APPARATUS FOR STONE FRAGMENTS REMOVAL**
VORRICHTUNG ZUR BESEITIGUNG VON STEINFRAGMENTEN
APPAREIL D'ÉLIMINATION DE FRAGMENTS DE CALCUL

(30) Priority: 28.01.2016 US 201615008533
(43) Date of publication of application: 29.09.2021
(62) Divisional of application: 17702290.2
(73) Proprietor: Gyrus ACMI, Inc. d/b/a Olympus Surgical Technologies America, Westborough, MA 01581 (US)
(72) Inventor: Fan, Tailin, Nashua, NH 03063 (US)
(74) Representative: Noack, Andreas

(56) References cited:
- EP-A1- 0 668 058
- WO-A1-2014/169923
- US-A- 5 320 599
- US-A1- 2014 200 570
- US-B2- 8 430 837

## Description

### FIELD

The embodiments of the present disclosure relate generally to a medical device or system for the removal of small stones or stone fragments from within the body's lumen or internal organ of a subject. More particularly, the embodiments of the present disclosure relate to a medical device or system for the removal of small stones or stone fragments from within the body's urinary system or renal system of a subject.

### BACKGROUND

Many stone extraction devices such as stents, retrieval assemblies, and coiled medical extraction devices can be used to entrap solid materials such as stone fragments, and drag them out from within the body lumen to remove them. Coiled medical extraction devices may also be used to prevent unwanted migration of stone fragments generated during a stone fragmentation procedure, and then safely and efficiently extract stone fragments from a patient's body.

It is also known that current methods of breaking up kidney stones may result in large amounts of small stone fragments scattered in the ureter or kidney. Standard baskets struggle to grasp these small stone fragments, and these large amounts of stone fragments mean longer procedures if they are to be removed independently. Any stone fragments left behind after the completion of the procedure can become nucleation points for new stone formation or may be painful to pass naturally.

US patent No. 7,122,017 discloses a variety of surgical instruments for forming a liquid jet, which are useful for performing a wide variety of surgical procedures. The disclosed invention provides surgical liquid jet instruments having a pressure lumen and an evacuation lumen, where the pressure lumen includes at least one nozzle for forming a liquid jet and where the evacuation lumen includes a jet-receiving opening for receiving the liquid jet when the instrument is in operation. The disclosed pressure lumen and the evacuation lumen of the surgical liquid jet instruments are constructed and positionable relative to each other so that the liquid comprising the liquid jet, and any tissue or material entrained by the liquid jet can be evacuated through the evacuation lumen without the need for an external source of suction. The disclosed invention also provides a variety of surgical liquid jet instruments that are constructed and configured specifically for use in a surrounding liquid environment or a surrounding gaseous environment. The patent also discloses a variety of surgical liquid jet instruments that are rotatably deployable from an undeployed position, for insertion into the body of a patient, to a deployed position, in which there is a separation distance between the liquid jet nozzle and the jet-receiving opening that defines a liquid jet path length. The patent further discloses surgical methods utilizing the surgical liquid jet instruments, and methods for forming components of the surgical liquid jet instruments.

US patent application publication No. 2015/0,119,645 discloses systems and methods for controlling pressure during percutaneous and endoscopic surgeries, including percutaneous renal procedures, endoscopic uterine procedures, transurethral endoscopic procedures for the bladder or prostate, or any percutaneous or endoscopic procedure. The disclosed system can include a sheath and/or endoscope each having an inflow port providing access to an inflow channel extending from the inflow port to a distal portion of the sheath, and an outflow port providing access to an outflow channel extending from the outflow port to the distal portion of the sheath. The disclosed sheath can also include a pressure sensor configured to generate a pressure measurement, and an electronic processor configured to control fluid through at least one of the inflow port and the outflow port based on the pressure measurement.

US patent application publication No. 2014/0,088,518A1 discloses a sheath assembly including sheath tubing, the sheath tubing having a main lumen and a secondary lumen that extends along the length of the main lumen, the main lumen being large enough to receive an endoscope.

US patent application publication No. 2005/0,261,705A1 discloses a medical retrieval basket device adapted to provide irrigation and suction to the region of the basket. The disclosed device includes a retrieval basket assembly having a sheath, an elongated member extending through the sheath, and a basket assembly coupled to the distal end of the elongated member, in a generally known arrangement. The disclosed retrieval basket assembly extends from a handle which provides suction and irrigation to, and through, the retrieval basket assembly. Fittings on the handle connect fluid and suction sources through fluid conduits within the handle to the sheath of the retrieval basket assembly. The disclosed retrieval basket assembly's sheath has an expandable distal end portion that improves the retrieval basket assembly's ability to capture debris from lithotripsy of a stone.

The US 2014/0200570 A1 discloses an instrument for applying thermal energy to tissue, wherein vapor is supplied to and evacuated from a treatment site through a probe. The US 5,320,599 discloses a drainage catheter with a pressure channel ending in a jet nozzle directed towards a receiving end of an evacuation channel. The WO 2014/16923 A1 discloses a multi-lumen catheter with three lumens. The EP 0 668 058 A1 discloses a treatment catheter having a distal opening for engaging tissue under treatment, and fluid channels for circulating treatment fluid along the engaged tissue. The US 8,430,837 discloses a thrombectomy catheter with a low pressure lumen for evacuating thrombi, and a high pressure lumen for breaking up large thrombi to facilitate evacuation.

### SUMMARY OF THE DISCLOSURE

The present invention relates to a medical device as defined in independent claim 1 and a system according to claim 7. Further embodiments of the invention are specified in the dependent claims. In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; and an outflow lumen also disposed within the elongate member, the outflow lumen having a proximal end, a distal end and a slanting receiving opening at the distal end of the outflow lumen; wherein the distal end of the inflow lumen and the distal end of the outflow lumen are configured to be connected such that the connecting portion surrounds both the exit opening of the inflow lumen and the receiving opening of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and at least one pressure sensor; wherein the distal end of the inflow lumen and the distal end of the outflow lumen are configured to be connected such that the connecting portion surrounds both the exit opening of the inflow lumen and the receiving opening of the outflow lumen. In one aspect, the at least pressure sensor is disposed at the distal end of the elongate member. In one aspect, the device comprises two pressure sensors. In one aspect, the two pressure sensors are both disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; and an outflow lumen also disposed within the elongate member, the outflow lumen having a proximal end, a distal end and a slanting receiving opening at the distal end of the outflow lumen; wherein the distal end of the inflow lumen and the distal end of the outflow lumen are configured to be connected such that the majority of the fluid from the exit opening of the inflow lumen directly flows into the receiving opening of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and at least one pressure sensor; wherein the distal end of the inflow lumen and the distal end of the outflow lumen are configured to be connected such that the majority of the fluid from the exit opening of the inflow lumen directly flows into the receiving opening of the outflow lumen. In one aspect, the at least pressure sensor is disposed at the distal end of the elongate member. In one aspect, the device comprises two pressure sensors. In one aspect, the two pressure sensors are both disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; and an outflow lumen also disposed within the elongate member, the outflow lumen having a proximal end, a distal end and a slanting receiving opening at the distal end of the outflow lumen; wherein the slanting exit opening of the inflow lumen and the slanting receiving opening of the outflow lumen are configured to face each other; and wherein the distal end of the inflow lumen and the distal end of the outflow lumen are configured to be connected such that the connecting portion surrounds both the slanting exit opening of the inflow lumen and the slanting receiving opening of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and at least one pressure sensor; wherein the slanting exit opening of the inflow lumen and the slanting receiving opening of the outflow lumen are configured to face each other; and wherein the distal end of the inflow lumen and the distal end of the outflow lumen are configured to be connected such that the connecting portion surrounds both the slanting exit opening of the inflow lumen and the slanting receiving opening of the outflow lumen. In one aspect, the at least pressure sensor is disposed at the distal end of the elongate member. In one aspect, the device comprises two pressure sensors. In one aspect, the two pressure sensors are both disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; at least one pressure sensor, the at least one pressure sensor configured to generate a pressure measurement when in operation; and a processing unit configured to control fluid flow through the inflow lumen or the outflow lumen or both based on the pressure measurement; wherein the distal end of the inflow lumen and the distal end of the outflow lumen are configured to be connected such that the connecting portion surrounds both the exit opening of the inflow lumen and the receiving opening of the outflow lumen. In one aspect, the at least pressure sensor is disposed at the distal end of the elongate member. In one aspect, the device comprises two pressure sensors. In one aspect, the two pressure sensors are both disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; at least one pressure sensor, the at least one pressure sensor configured to generate a pressure measurement when in operation; and a processing unit configured to control fluid flow through the inflow lumen or the outflow lumen or both based on the pressure measurement; wherein the slanting exit opening of the inflow lumen and the slanting receiving opening of the outflow lumen are configured to face each other; and wherein the distal end of the inflow lumen and the distal end of the outflow lumen are configured to be connected such that the connecting portion surrounds both the slanting exit opening of the inflow lumen and the slanting receiving opening of the outflow lumen. In one aspect, the at least pressure sensor is disposed at the distal end of the elongate member. In one aspect, the device comprises two pressure sensors. In one aspect, the two pressure sensors are both disposed at the distal end of the elongate member.

In one aspect, the present disclosure provides a system for removing intracorporeal small stones or stone fragments during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; at least one pressure sensor, the at least pressure sensor configured to generate a pressure measurement when in operation; and a processing unit configured to control fluid flow through the inflow lumen or the outflow lumen or both based on the pressure measurement; wherein the distal end of the inflow lumen and the distal end of the outflow lumen are configured to be connected such that the connecting portion surrounds both the exit opening of the inflow lumen and the receiving opening of the outflow lumen. In one aspect, the at least pressure sensor is disposed at the distal end of the sheath. In one aspect, the system comprises two pressure sensors disposed at the distal end of the sheath. In one aspect, the system further comprises an endoscope having a working channel and an illumination channel wherein the sheath may be inserted into the body lumen or internal organ through the endoscope.

In one aspect, the present disclosure provides a an exemplary method of removing small stone fragments from within the body lumen or internal organ of a subject, the method comprising providing a medical device or system or variations thereof as described herein; inserting the medical device or system into a body lumen or internal organ; irrigating the inflow lumen of the medical device or system with a liquid; and aspirating the liquid out of the outflow lumen of the medical device or system to remove the small stone fragments from within the body lumen or internal organ of the subject.

### BRIEF DESCRIPTION OF THE DRAWINGS

FIG. 1 is an illustrative representation of a medical device in accordance with the present invention wherein the slanting exit opening of the inflow lumen and the slanting receiving opening of the outflow lumen of the medical device are smoothly curved and they are also the mirror images of each other.
FIG. 2 is an illustrative representation of a medical device in accordance with the present invention wherein the slanting exit opening of the inflow lumen and the slanting receiving opening of the outflow lumen of the medical device are substantially straight cut and they are also the mirror images of each other.
FIG. 3 is an illustrative representation of a device in accordance with the embodiment of FIG. 1, wherein the two pressure sensors are more clearly shown to be at the distal end of the device.
FIG. 4 is a cut-off and perspective illustration of a medical device in accordance with an aspect of the present disclosure wherein the exit opening of the inflow lumen is completely merged with the outflow lumen.

### DETAILED DESCRIPTION

The explanations and illustrations presented herein are intended to acquaint others skilled in the art with the disclosure, its principles, and its practical applications. Those skilled in the art may adapt and apply the disclosure in numerous forms, as may be best suited to the requirements of a particular use. The specific embodiments of the present disclosure as set forth are not intended to be exhaustive or limiting of the invention. The scope of the invention should be determined not with reference to the above description, but should be determined with reference to the appended claims.

The terms "one aspect", "one embodiment", "an embodiment", "another embodiment", "some embodiments", "other embodiments", and similar expressions indicate that the embodiment or embodiments described may include a particular feature, structure, or characteristic, but every embodiment may not necessarily include the particular feature, structure, or characteristic. Moreover, such phrases are not necessarily referring to the same embodiment. Furthermore, when a particular feature, structure, or characteristic is described in connection with an embodiment, it would be within the knowledge of one skilled in the art to incorporate such feature, structure, or characteristic into other embodiments, whether or not explicitly described, unless clearly stated to the contrary. That is, the various individual elements described below, even if not explicitly shown in a particular combination, are nevertheless contemplated as being combinable with each other to form other additional embodiments or to complement and/or enrich the described embodiment or embodiments, as would be understood by one of ordinary skill in the art.

The articles "a", "an" and "the"" are used herein to refer to one or to more than one (i.e. to at least one) of the grammatical object of the article unless otherwise clearly indicated by contrast. By way of example, "an element" means one element or more than one element.

The term "including" is used herein to mean, and is used interchangeably with, the phrase "including but not limited to". The term "or" is used herein to mean, and is used interchangeably with, the term "and/or", unless context clearly indicates otherwise.

The term "such as" is used herein to mean, and is used interchangeably, with the phrase "such as but not limited to". Unless specifically stated or obvious from context, as used herein, the term "about" is understood as within a range of normal acceptance in the art, for example within standard deviations of the mean.

The term "proximal" is herein used to mean a position or direction closest to a user of the device and is in a position or direction opposite to the term "distal".

The term "distal" is herein used to mean a position or direction furthest away from a user of the device and is a position or direction opposite to the term "proximal".

All numeric values are herein assumed to be modified by the term "about" whether or not explicitly indicated. The term "about", in the context of numeric values, generally refers to a range of numbers that one of skill in the art would consider equivalent to the recited value (i.e., having the same function or result). In many instances, the term "about" may include numbers that are rounded to the nearest significant figure. Other uses of the term "about" (i.e., in a context other than numeric values) may be assumed to have their ordinary and customary definition(s), as understood from and consistent with the context of the specification, unless otherwise specified. Even more specifically, "about" can be understood as within 10%, 9%, 8%, 7%, 6%, 5%, 4%, 3%, 2%, 1%, 0.5%, 0.1 %, 0.05%, or 0.01 % of the stated value.

Any feature, structure, or step as disclosed herein can be replaced or combined with any other feature, structure, or step disclosed herein. Further, for purpose of summarizing the disclosure, certain aspects, advantages, and features have been described herein. It is to be understood that not necessarily any or all such advantages are achieved in accordance with any particular embodiment of the disclosure herein.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; and an outflow lumen also disposed within the elongate member, the outflow lumen having a proximal end, a distal end and a slanting receiving opening at the distal end of the outflow lumen; wherein the distal end of the inflow lumen and the distal end of the outflow lumen are configured to be connected such that the connecting portion surrounds both the exit opening of the inflow lumen and the receiving opening of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and at least one pressure sensor; wherein the distal end of the inflow lumen and the distal end of the outflow lumen are configured to be connected such that the connecting portion surrounds both the exit opening of the inflow lumen and the receiving opening of the outflow lumen. In one aspect, the at least one pressure sensor is disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and two pressure sensors; wherein the distal end of the inflow lumen and the distal end of the outflow lumen are configured to be connected such that the connecting portion surrounds both the exit opening of the inflow lumen and the receiving opening of the outflow lumen. In one aspect, one of the two pressure sensors is configured to generate a pressure measurement for the inflow liquid while the other pressure sensor is configured to generate a pressure measurement for the outflow liquid when in operation. In one aspect, both of the two pressure sensors are disposed at the distal end of the elongate member. In one aspect, both of the two pressure sensors are disposed at the proximal end of the elongate member. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; and an outflow lumen also disposed within the elongate member, the outflow lumen having a proximal end, a distal end and a slanting receiving opening at the distal end of the outflow lumen; wherein the distal end of the inflow lumen and the distal end of the outflow lumen are configured to be connected such that the majority of the fluid from the exit opening of the inflow lumen directly flows into the receiving opening of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and at least one pressure sensor; wherein the distal end of the inflow lumen and the distal end of the outflow lumen are configured to be connected such that the majority of the fluid from the exit opening of the inflow lumen directly flows into the receiving opening of the outflow lumen. In one aspect, the at least one pressure sensor is disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and two pressure sensors; wherein the distal end of the inflow lumen and the distal end of the outflow lumen are configured to be connected such that the majority of the fluid from the exit opening of the inflow lumen directly flows into the receiving opening of the outflow lumen. In one aspect, one of the two pressure sensors is configured to generate a pressure measurement for the inflow liquid while the other pressure sensor is configured to generate a pressure measurement for the outflow liquid when in operation. In one aspect, both of the two pressure sensors are disposed at the distal end of the elongate member. In one aspect, both of the two pressure sensors are disposed at the proximal end of the elongate member. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; and an outflow lumen also disposed within the elongate member, the outflow lumen having a proximal end, a distal end and a slanting receiving opening at the distal end of the outflow lumen; wherein the slanting exit opening of the inflow lumen and the slanting receiving opening of the outflow lumen are configured to face each other; and the distal end of the inflow lumen and the distal end of the outflow lumen are configured to be connected such that the connecting portion surrounds both the slanting exit opening of the inflow lumen and the slanting receiving opening of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and at least one pressure sensor; wherein the slanting exit opening of the inflow lumen and the slanting receiving opening of the outflow lumen are configured to face each other; and the distal end of the inflow lumen and the distal end of the outflow lumen are configured to be connected such that the connecting portion surrounds both the slanting exit opening of the inflow lumen and the slanting receiving opening of the outflow lumen. In one aspect, the at least one pressure sensor is disposed at the distal end of the elongate member.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; and two pressure sensors; wherein the slanting exit opening of the inflow lumen and the slanting receiving opening of the outflow lumen are configured to face each other; and the distal end of the inflow lumen and the distal end of the outflow lumen are configured to be connected such that the connecting portion surrounds both the slanting exit opening of the inflow lumen and the slanting receiving opening of the outflow lumen. In one aspect, one of the two pressure sensors is configured to generate a pressure measurement for the inflow liquid while the other pressure sensor is configured to generate a pressure measurement for the outflow liquid when in operation. In one aspect, both of the two pressure sensors are disposed at the distal end of the elongate member. In one aspect, both of the two pressure sensors are disposed at the proximal end of the elongate member. In one aspect, one pressure sensor is disposed at the distal end of the inflow lumen, and the other pressure sensor at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; at least one pressure sensor, the at least pressure sensor configured to generate a pressure measurement when in operation; and a processing unit configured to control fluid flow through the inflow lumen or the outflow lumen or both based on the pressure measurement; wherein the distal end of the inflow lumen and the distal end of the outflow lumen are configured to be connected such that the connecting portion surrounds both the exit opening of the inflow lumen and the receiving opening of the outflow lumen. In one aspect, the at least one pressure sensor is disposed at the distal end of the elongate member. In one aspect, the device comprises two pressure sensors. In one aspect, one of the two pressure sensors is configured to generate a pressure measurement for the inflow liquid while the other pressure sensor is configured to generate a pressure measurement for the outflow liquid when in operation. In one aspect, both of the two pressure sensors are disposed at the distal end of the elongate member. In one aspect, both of the two pressure sensors are disposed at the proximal end of the elongate member. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; at least one pressure sensor, the at least one pressure sensor configured to generate a pressure measurement when in operation; and a processing unit configured to control fluid flow through the inflow lumen or the outflow lumen or both based on the pressure measurement; wherein the slanting exit opening of the inflow lumen and the slanting receiving opening of the outflow lumen are configured to face each other; and the distal end of the inflow lumen and the distal end of the outflow lumen are configured to be connected such that the connecting portion surrounds both the slanting exit opening of the inflow lumen and the slanting receiving opening of the outflow lumen. In one aspect, the exit opening and the receiving opening are substantially identical with respect to their cross-section shape and/or configuration. In one aspect, the exit opening is a mirror image of the receiving opening with respect to a plane along the longitudinal axis of the medical device. In one aspect, the at least one pressure sensor is disposed at the distal end of the elongate member. In one aspect, the device comprises two pressure sensors. In one aspect, one of the two pressure sensors is configured to generate a pressure measurement for the inflow liquid while the other pressure sensor is configured to generate a pressure measurement for the outflow liquid when in operation. In one aspect, both of the two pressure sensors are disposed at the distal end of the elongate member. In one aspect, both of the two pressure sensors are disposed at the proximal end of the elongate member. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen.

In one aspect, the present disclosure provides a medical device comprising an elongate member having a distal end and a proximal end; an inflow lumen disposed within the elongate member, the inflow lumen having a proximal end, a distal end and a slanting exit opening at the distal end of the inflow lumen; an outflow lumen also disposed within the elongate member, the outflow lumen having a proximal end, a distal end and a slanting receiving opening at the distal end of the outflow lumen; at least one pressure sensor configured to generate a pressure measurement when in operation; and a processing unit configured to control fluid flow through the inflow lumen or the outflow lumen or both based on the pressure measurement; wherein the distal end of the inflow lumen and the distal end of the outflow lumen are configured to be connected such that the majority of the fluid from the exit opening of the inflow lumen directly flows into the receiving opening of the outflow lumen. In one aspect, the at least one pressure sensor is disposed at the distal end of the elongate member. In one aspect, the device comprises two pressure sensors. In one aspect, one of the two pressure sensors is configured to generate a pressure measurement for the inflow liquid while the other pressure sensor is configured to generate a pressure measurement for the outflow liquid when in operation. In one aspect, both of the two pressure sensors are disposed at the distal end of the elongate member. In one aspect, both of the two pressure sensors are disposed at the proximal end of the elongate member. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen.

In one aspect, the present disclosure provides a system for removing intracorporeal stones during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; at least one pressure sensor, the at least pressure sensor configured to generate a pressure measurement when in operation; and a processing unit configured to control fluid flow through the inflow lumen or the outflow lumen or both based on the pressure measurement; wherein the distal end of the inflow lumen and the distal end of the outflow lumen are configured to be connected such that the connecting portion surrounds both the exit opening of the inflow lumen and the receiving opening of the outflow lumen. In one aspect, the at least one pressure sensor is disposed at the distal end of the sheath.

In one aspect, the present disclosure provides a system for removing intracorporeal small stones or stone fragments during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; two pressure sensors, one pressure sensor configured to generate a pressure measurement for the inflow liquid when in operation; the other pressure sensor configured to generate a pressure measurement for the outflow liquid, and a processing unit configured to control fluid flow through the inflow lumen and the outflow lumen based on the two pressure measurements; and a processing unit configured to control fluid flow through the inflow lumen or the outflow lumen or both based on the pressure measurement; wherein the distal end of the inflow lumen and the distal end of the outflow lumen are configured to be connected such that the connecting portion surrounds both the exit opening of the inflow lumen and the receiving opening of the outflow lumen. In one aspect, both of the two pressure sensors are disposed at the distal end of the sheath. In one aspect, both of the two pressure sensors are disposed at the proximal end of the sheath. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen. In one aspect, the system further comprises an endoscope having a working channel and an illumination channel wherein the sheath may be inserted into the body lumen or internal organ through the endoscope.

In one aspect, the present disclosure provides a system for removing intracorporeal stones during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; at least one pressure sensor, the at least pressure sensor configured to generate a pressure measurement when in operation; and a processing unit configured to control fluid flow through the inflow lumen or the outflow lumen or both based on the pressure measurement; wherein the distal end of the inflow lumen and the distal end of the outflow lumen are configured to be connected such that the majority of the fluid from the exit opening of the inflow lumen directly flows into the receiving opening of the outflow lumen. In one aspect, the at least one pressure sensor is disposed at the distal end of the sheath.

In one aspect, the present disclosure provides a system for removing intracorporeal small stones or stone fragments during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; two pressure sensors, one pressure sensor configured to generate a pressure measurement for the inflow liquid when in operation; the other pressure sensor configured to generate a pressure measurement for the outflow liquid, and a processing unit configured to control fluid flow through the inflow lumen and the outflow lumen based on the two pressure measurements; and a processing unit configured to control fluid flow through the inflow lumen or the outflow lumen or both based on the pressure measurement; wherein the distal end of the inflow lumen and the distal end of the outflow lumen are configured to be connected such that the majority of the fluid from the exit opening of the inflow lumen directly flows into the receiving opening of the outflow lumen. In one aspect, both of the two pressure sensors are disposed at the distal end of the sheath. In one aspect, both of the two pressure sensors are disposed at the proximal end of the sheath. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen. In one aspect, the system further comprises an endoscope having a working channel and an illumination channel wherein the sheath may be inserted into the body lumen or internal organ through the endoscope.

In one aspect, the present disclosure provides a system for removing intracorporeal stones during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; at least one pressure sensor, the at least pressure sensor configured to generate a pressure measurement when in operation; and a processing unit configured to control fluid flow through the inflow lumen or the outflow lumen or both based on the pressure measurement; wherein the slanting exit opening of the inflow lumen and the slanting receiving opening of the outflow lumen are configured to face each other; and the distal end of the inflow lumen and the distal end of the outflow lumen are configured to be connected such that the connecting portion surrounds both the slanting exit opening of the inflow lumen and the slanting receiving opening of the outflow lumen. In one aspect, the at least one pressure sensor is disposed at the distal end of the sheath. In one aspect, the exit opening and the receiving opening are substantially identical with respect to their cross-section shape and/or configuration. In one aspect, the exit opening is a mirror image of the receiving opening with respect to a plane along the longitudinal axis of the medical device.

In one aspect, the present disclosure provides a system for removing intracorporeal small stones or stone fragments during an endoscopic or medical operation, the system comprising a sheath having a distal end and a proximal end; an inflow lumen disposed within the sheath, the inflow lumen having a proximal end, a distal end and a slanting exit opening at the distal end of the inflow lumen; an outflow lumen also disposed within the sheath, the outflow lumen having a proximal end, a distal end and a slanting receiving opening at the distal end of the outflow lumen; two pressure sensors, one pressure sensor configured to generate a pressure measurement for the inflow liquid when in operation; the other pressure sensor configured to generate a pressure measurement for the outflow liquid, and a processing unit configured to control fluid flow through the inflow lumen and the outflow lumen based on the two pressure measurements; and a processing unit configured to control fluid flow through the inflow lumen or the outflow lumen or both based on the pressure measurement; wherein the slanting exit opening of the inflow lumen and the slanting receiving opening of the outflow lumen are configured to face each other; and the distal end of the inflow lumen and the distal end of the outflow lumen are configured to be connected such that the connecting portion surrounds both the slanting exit opening of the inflow lumen and the slanting receiving opening of the outflow lumen. In one aspect, both of the two pressure sensors are disposed at the distal end of the sheath. In one aspect, both of the two pressure sensors are disposed at the proximal end of the sheath. In one aspect, one pressure sensor is disposed at the distal end of the inflow lumen, and the other pressure sensor disposed at the distal end of the outflow lumen. In one aspect, one pressure sensor is disposed at the proximal end of the inflow lumen, and the other pressure sensor disposed at the distal end of the outflow lumen. In one aspect, one pressure sensor is disposed at the proximal end of the inflow lumen, and the other pressure sensor disposed at the proximal end of the outflow lumen. In one aspect, the system further comprises an endoscope having a working channel and an illumination channel wherein the sheath may be inserted into the body lumen or internal organ through the endoscope.

In some of the above embodiments, both of the inflow lumen and the outflow lumen may be configured to be substantially parallel to the longitudinal axis of the medical device. In some of the above embodiments, both of the inflow lumen and the outflow lumen may be configured to have substantially identical diameter and to be substantially linear along the longitudinal axis of the medical device. In some of the above embodiments, the cross sections of both the inflow lumen and the outflow lumen may be configured to be substantially circular. In some of the above embodiments, the inflow lumen and the outflow lumen may be configured to be substantially symmetrical along the longitudinal axis of the device. In some of the above embodiments, the inflow lumen may be substantially cylindrical. In some of the above embodiments, the inflow lumen may be substantially tubular. In some of the above embodiments, the inflow lumen may be substantially cylindrical with substantially identical cross section diameter along the whole inflow lumen length. In some of the above embodiments, the outflow lumen may be substantially cylindrical. In some of the above embodiments, the outflow lumen may be substantially tubular. In some of the above embodiments, the outflow lumen may be substantially cylindrical along the whole outflow lumen length. In some of the above embodiments, the cross-sectional diameter of the cylindrical inflow lumen is in the range of about 0.5 mm to about 2.5 mm. In some of the above embodiments, the cross sectional diameter of the cylindrical outflow lumen is in the range of about 0.5 mm to about 2.5 mm. In some of the above embodiments, the inflow lumen is separated from the outflow lumen just by a wall of the thickness of about 0.5 mm to about 1.0 mm.

In some of the above embodiments, the slanting exit opening is a mirror image of the slanting receiving opening of the outflow lumen with respect to a plane along the longitudinal axis of the device. The liquid flowing out of the slanting exit opening of the inflow lumen produces a micro lower pressure region such that small stones are sucked into the lower pressure region and then taken out of this region through the slanting receiving opening of the outflow lumen. The slanting exit opening of the inflow lumen may be made through known methods such as laser technologies. For example, the diameter of the slanting exit opening may be equal to the full diameter of the inflow lumen at the start of the slant to about half of the diameter at the end of the exit slant. Similarly, the diameter of the slanting receiving opening of the outflow may be equal to the full diameter of outflow lumen at the start of the slant to about half of the diameter at the end of the receiving slant. In some embodiments, the slanting exit opening may cover a length of about 1 mm to about 5 mm along the longitudinal axis of the device. In some embodiments, the slanting receiving opening may cover a length of about 1 mm to about 5 mm along the longitudinal axis of the device.

In some of the above embodiments, the whole medical device may be made from polymeric materials or their mixtures such as polycarboxylic acid polymers and copolymers such as polyacrylic acids; acetal polymers and copolymers; acrylate and methacrylate polymers and copolymers such as n-butyl methacrylate; cellulosic polymers and copolymers such as cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides and polyether block amides, polyamidimides, polyesterimides, and polyetherimides; polysulfone polymers and copolymers such as polyarylsulfones and polyethersulfones; polyamide polymers and copolymers such as nylon 66, nylon 12, polycaprolactams and polyacrylamides; resins such as alkyd resins, phenolic resins, urea resins, melamine resins, epoxy resins, allyl resins and epoxide resins; polycarbonates; polyacrylonitriles; polyvinylpyrrolidones; polymers and copolymers of vinyl monomers such as polyvinyl alcohols, polyvinyl halides such as polyvinyl chlorides, ethylene-vinyl acetate copolymers (EVA), polyvinylidene chlorides, polyvinyl ethers such as polyvinyl methyl ethers, polystyrenes, styrene-maleic anhydride copolymers, vinylaromatic-olefin copolymers such as styrene-butadiene copolymers, styrene-ethylene-butylene copolymers (e.g., a polystyrene-polyethylene/butylene-polystyrene (SEBS) copolymer, available as Kraton^{®} G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene and polystyrene-polyisobutylene-polystyrene triblock copolymers), polyvinyl ketones, polyvinylcarbazoles, and polyvinyl esters such as polyvinyl acetates; polybenzimidazoles; ethylene-methacrylic acid copolymers and ethylene-acrylic acid copolymers, where some of the acid groups can be neutralized with either zinc or sodium ions (commonly known as ionomers); polyalkyl oxide polymers and copolymers including polyethylene oxides (PEO); polyesters including polyethylene terephthalates and aliphatic polyesters such as polymers and copolymers of lactide (which includes lactic acid as well as d-,l- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of poly(lactic acid) and poly(caprolactone)); polyether polymers and copolymers such as polyarylethers (polyphenylene ethers, polyether ketones, polyether ether ketones); polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; thermoplastic polyurethanes (TPU); elastomers such as elastomeric polyurethanes and polyurethane copolymers (including block and random copolymers that are polyether based, polyester based, polycarbonate based, aliphatic based, aromatic based and mixtures thereof; examples of commercially available polyurethane copolymers include Bionate^{®}, Carbothane^{®}, Tecoflex^{®}, Tecothane^{®}, Tecophilic^{®}, Tecoplast^{®}, Pellethane^{®}, Chronothane^{®} and Chronoflex^{®}); p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as copolymers of the above.

In some of the above embodiments, the whole device may also be made from suitable metals such as nitinol and stainless steel. In some of the above embodiments, however, the inflow lumen section and the outflow section may be made from different polymeric or metallic materials. In some of the above embodiments, both the inflow lumen walls and the outflow lumen walls may be strengthened by metal spirals for varying pressure needs. In some of the above embodiments, only the connecting portions may be made from metallic materials, and the rest made of polymeric materials. In some of the above embodiments, the connecting portions may be made of polymeric materials reinforced by metal spirals.

In some of the above embodiments, the overall length of the medical device may vary in accordance with its intended application. A relatively longer device may be advantageous for retrieving solid materials or stones deep inside the body of a subject if employed independently. For example, the overall length of the medical device may be in the range of from about 1.0 to about 3.0 inches, or preferably from about 1.5 to about 2.0 inches. The medical device may be configured to be flexible along at least portion of its length so that it may bend as it is advanced through a tortuous body lumen. The overall outer diameter of the medical device should be configured to be less than about 4 mm, or preferably less than about 3.5 mm in order to go through a ureter access sheath such as a 12/14 Fr access sheath. The medical device should generally be configured to have a configuration, dimensions or material properties that allow its longitudinal movement within an external access sheath for extension and/or retraction into and out of the external access sheath if necessary. (1 inch ≅ 25.4 mm and 1 Fr ≅ 0.33 mm)

In some of the above embodiments, the connecting portions of the medical device are configured to direct the liquid flowing from the exit opening of the inflow lumen towards the receiving opening of the outflow in a most efficient manner. They made be made from the same polymeric materials as previously described for both the inflow lumen and the outflow lumen. They may be also made as separate parts, followed by attachment to the openings by various known mechanisms such as a screw fit, a snap fit, a friction fit, or by suitable adhesives. For example, they may be made of thin wall metal materials such as stainless steel and nitinol. The connection portions may be made into different shapes or sizes based on the specific need of the medical device. For example, the connection portion may be configured as a semicircle piece with grooves matching both the shape of the exit opening of the inflow lumen and the shape of the receiving opening of the outflow lumen for better hydraulic effects.

In some of the above embodiments, the proximal end of the inflow lumen is configured to be connectable to a source of liquid under pressure supplied by, for example, a pressure pump or liquid dispenser. The liquid pressure supplied by the pump or dispenser is variably controllable or adjustable by an operator of the device. The liquid pressure supplied by the pump or dispenser can also be automatically controlled or adjusted by a processor, processing unit, and/or a controller. In some embodiments, the proximal end of the outflow lumen is configured to be connectable to an external source of suction such as a vacuum pump or aspirator to provide the suction driving force required for evacuating the small stones from the surgical field via the slanting receiving opening. In the above embodiments, the connectable mechanism may be any known methods such as screw fit, a snap fit, and adhesive attachment.

In some embodiments, the pressure sensor can be any type of pressure transducer, pressure transmitter, pressure sender, pressure indicator, piezometer or manometer. In some embodiments, more than one pressure sensor can be placed at the tip of the device, for example, two or three pressure sensor can be placed at the tip of the device. In some embodiment, other sensors can also be placed at the tip of the device if necessary or desired, for example, temperature sensors, or optical sensors.

In the embodiments wherein a processing unit is required, the processing unit may be a general purpose processor device, a digital signal process (DSP), an application specific integrated circuit, a field programmable gate array, or other programmable logic device, discrete gate or transistor logic, discrete hardware components or any combinations thereof. A general purpose processor device can be a microprocessor, but in the alternative, the processor device can be a controller, microcontroller, or state machine, combinations of the same, or the like. A processor device can include electrical circuitry configured to process computer-executable instructions. In another embodiment, a processor device includes an FPGA or other programmable device that performs logic operations without processing computer-executable instructions. A processor device can also be implemented as a combination of computing devices, e.g., a combination of a DSP and a microprocessor, a plurality of microprocessors, one or more microprocessors in conjunction with a DSP core, or any other such configuration. Although described herein primarily with respect to digital technology, a processor device may also include primarily analog components. A computing environment can include any type of computer system, including, but not limited to, a computer system based on a microprocessor, a mainframe computer, a digital signal processor, a portable computing device, a device controller, or a computational engine within an appliance.

In some embodiments, the medical device may be made reusable. In some embodiments, the medical device may be made disposable. In some embodiments, the medical device is preferably configured to be disposable after a single use

In some embodiments, the medical device can include additional channels or lumens for illumination and/or other surgical purposes. For example, these additional channels may be used for a surgical instrument such as a grasper, a stone basket, laser fibers or the like.

In an embodiment, the medical device described herein may be used in lithotripsy or ureteroscopy to remove small kidney stones in the body of a patient. Lithotripsy is a medical procedure that uses energy in various forms such as acoustic shock waves, pneumatic pulsation, electro-hydraulic shock waves, or laser beams, to pulverize urinary calculi such as kidney stones. The force of the energy, when applied either extracorporeally or intracorporeally, breaks the stones down into smaller fragments that may be extracted from the body, or allowed to pass from the body, for example, through urination. In an embodiment, the solid materials to be removed are not limited to just small kidney stones. They may include small gallbladder stones, uric acid stones, or other small solid materials.

In one aspect, the present disclosure provides an exemplary method of capturing and removing small stones or stone fragments from within the body lumen or internal organ of a subject, the method comprising providing a medical device or system or variations thereof as described herein; irrigating the inflow lumen of the medical device or system with a liquid; and aspirating the liquid out of the outflow lumen of the medical device or system to remove the small stone fragments from within the body lumen or internal organ of the subject.

In one aspect, the present disclosure provides exemplary methods of using the medical device described herein to remove small stones or other small solid materials located in the bladder, ureter, kidney, or other body organs. In one aspect, the medical device is particularly suitable for the removal of stone fragments of less than 1 mm in diameter. The medical device may be inserted through the urethra of a subject, or percutaneously. The medical device may be used in any location of the body in which a passageway or orifice has unwanted small solid materials to be removed.

The medical device may be advanced to a treatment site through various ways. For example, the medical device may be advanced to a targeted location over a guidewire. In an embodiment, the medical device may be advanced to the targeted location by means of an imaging device. The medical device may also be advanced to the treatment site through an access sheath or any other suitable means known in the art.

The medical device may be used in conjunction with an endoscope or any other type of intrcorporeal device known in the art. The endoscope may be inserted through a body lumen into a treatment site in any conventional manner. Once the endoscope is properly positioned adjacent to the treatment site, the medical device may be led through an access port of the endoscope to get access to the targeted region for capture and removal of stones.

It will be apparent to the skilled addressee that many modifications, variants and improvements are possible within the ambit of the invention defined herein. For example, a device in accordance with some embodiments as described may be employed during the extracorporeal procedure.

The principles of the present disclosure may be better understood with reference to the drawings and the accompanying descriptions, wherein like reference numerals have been used throughout to designate identical or similar elements. It should be understood that these drawings are not necessarily are drawn to scale. They are presented just for illustrative purposes only, and are not intended to limit the scope of the disclosure. Examples of materials, dimensions, and constructions are included for some elements. Those of ordinary skill in the art should understand that many of the examples provided have suitable alternatives and these alternatives should also be considered within the scope of this disclosure. Moreover, certain terminology is used herein for convenience only and is not to be taken as a limitation on the present disclosure.

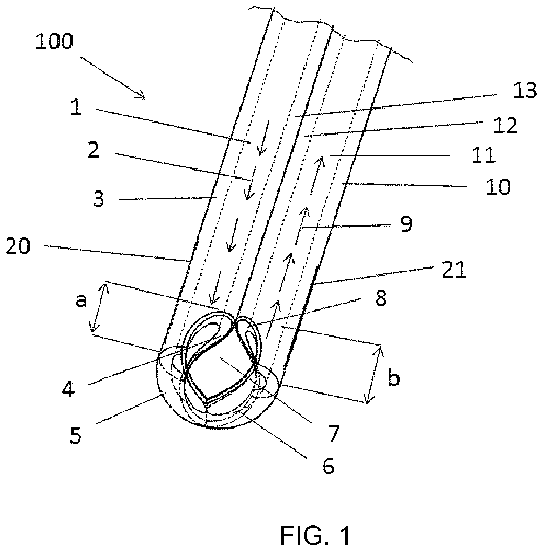

FIG. 1 illustrates a medical device 100 in accordance with one aspect of the present disclosure wherein the exit opening of the inflow lumen and the receiving opening of the outflow lumen of the medical device are smoothly curved and substantially symmetrical along the central longitudinal plane of the device. The medical device 100 of FIG. 1 comprises an inflow lumen section and an outflow lumen section connected at the tip or distal end of the medical device. The inflow lumen section is shown by arrows **2** pointing towards the tip or distal end of the device while the outflow lumen section is marked by arrows **9** moving away from the tip or distal end towards the proximal end (not shown) of the device. The inflow lumen section includes an inflow lumen **1**, an outer wall **3**, an inner wall **13**, an exit opening **4**, an exit connecting portion **5**, and a pressure sensor **20** (inflow pressure sensor) disposed at the distal end of the inflow section. The outflow lumen section includes a receiving connecting portion **6**, a receiving opening **8**, an outflow lumen **11,** an outer wall **10**, an inner wall **12**, and a second pressure sensor **21** (outflow pressure sensor) disposed at the distal end of the outflow lumen. As already indicated above, the arrows **2** of FIG. 1 show the direction in which the inflow liquid or irrigation liquid flows, while the arrows **9** show the direction in which the outflow liquid flows when the medical device is in operation. FIG. 1 further shows an open space **7** where the exit opening of the inflow lumen meets the receiving opening of the outflow lumen.

The inflow lumen **1** as shown in FIG. 1 is substantially linear in its longitudinal axis and circular in shape for its cross-section. However, it will be understood that it may have any desirable cross-sectional shape and/or configuration as may be expeditious for particular surgical application or convenient for manufacturing purpose. The diameter of the inflow lumen **1** may be from about 0.5 mm to about 2.5 mm. Preferably, it may be from about 1.0 mm to about 2.5 mm. More preferably, it may be from about 1.0 mm to about 1.5 mm.

The outer wall **3** of the inflow lumen may be configured to have identical wall thickness along the whole longitudinal length of the inflow lumen. It may also be configured to have varying wall thicknesses along the longitudinal length. Similarly, the inner wall **13** of the inflow lumen may be configured to have identical wall thickness along the whole longitudinal length. It may also be configured to have varying wall thicknesses along the longitudinal length. The thickness of both walls may be in the range of about 0.1 mm to about 1.0 mm. Preferably, the thickness of both walls may be in the range of about 0.2 mm to about 0.5 mm. More preferably, the thickness of both walls may be in the range of about 0.2 mm to about 0.4 mm. In addition, the outer wall **3** and the inner wall **13** of the inflow lumen may have the same or different wall thickness, even though it may be preferred that both have substantially identical thickness.

The exit opening **4** of the inflow lumen as shown in FIG. 1 has a smooth curved shape. However, it will be understood that it may have any desirable cross-sectional shape and/or configuration as may be expeditious for particular surgical application or convenient for manufacturing purpose. The exit opening 4 of the inflow lumen as shown tapers from a substantially circular cross-section shape at the beginning of the opening to a semi-circle cross-section shape towards the end of the opening. The end of the opening may also be configured to be substantially smaller than a semi-circle cross-section shape. The tapering may also be in straight cut, wavy cut, smooth curved cut, or irregular smooth curved shape. The opening 4 may be configured to have a length of about 1 mm to about 5 mm along the longitudinal axis (the distance "a" as shown in FIG. 1). All these shapes/configurations may be achieved through conventional methods/techniques such as laser related techniques.

The exit connecting portion **5** may be configured to have similar cross-sectional shape or configuration as that of the exit opening **4.** However, it will be understood that it may have any desirable cross-sectional shape and/or configuration as may be expeditious for particular surgical application or convenient for manufacturing purpose. It may also be configured to have the substantially the same longitudinal length as that of the exit opening **4**, which may be in the range of about 1 mm to about 5 mm. It can also have a different longitudinal length from that of the exit opening **4.** The exit connecting portion **5** may also be combined with the receiving connecting portion **6** of the outflow lumen to form one piece for the convenience of manufacturing.

The pressure sensor **20** of the inflow lumen (inflow pressure sensor) as shown in FIG. 1 is disposed at the distal end of the inflow section. It may also be disposed at the proximal end of the inflow lumen (not shown). It serves to provide information to the processing unit and/or controller (not shown) to control and/or monitor the fluid pressure flowing inside the inflow lumen. It may also be configured to serve to monitor the pressure of the kidney for the safety of the subject under operation. For example, it is known that the intrarenal irrigation pressure during an endoscopic procedure should not exceed 30 cm water in order to prevent pyelovenous backflow which could cause a series of medical complications if that happens. Consequently, it is critical that the intrarenal pressure should be maintained at less than about 30 cm water for such a procedure. It is recognized that it will be safer to monitor the intrarenal pressure by pressure sensors directly placed at the tip of the device. Consequently, it is preferred to place the inflow sensor at the distal end of the inflow lumen. It should also be understood though that the irrigation liquid pressure in the inflow lumen should be higher than that of the intrarenal irrigation pressure during such an operation. The irrigation liquid pressure in the inflow lumen is determined by its flow rate and the inflow lumen internal diameter, and the inflow rate should be sufficient to move the stone fragments at their depository site. This may all be achieved through the communications between the sensor and the processing unit and/or the controller.

The inflow pressure sensor **20** can be any type of pressure transducer, pressure transmitter, pressure sender, pressure indicator, piezometer or manometer. A preferred one is a low pressure sensor such as the magneto-impedance multilayer type as described in Fernandez et al at Nanoscale Research Letters, 2012, (7) 230. For example, the pressure sensor can be made from an alloy of iron, titanium, copper and nickel, with a dimension of about 10 mm in length, about 0.5 mm in width, and about 1.3 µm in thickness. This type of pressure sensor may be attached at the very tip of the device or the outer wall of the distal end of the inflow lumen. This type of pressure sensor is highly desirable due to its small size, low cost, remote sensing, ease for implanting, and temporary deployment. Moreover, the inflow pressure sensor may be configured to be used as an active pressure sensing device, co-deployed with other microelectronics to communicate the intrarenal pressure to a processing unit and/or controller outside a patient's body by coated thin wire conductor or by known wireless means. It may also be configured to be used as a passive pressure sensor such as a magnet sensor which could alter a magnetic field generated outside a patient's body by a field generator/monitor, and then be sensed as pressure signal.

The inflow lumen **2** and its walls **3** and **13** may be made from known polymeric materials such as silicone, polyurethane, and polytetrafluoroethylene as previously described. The inflow lumen walls **3** and **13** can be strengthened by metal spirals for varying pressure needs. In some preferred aspects, the whole inflow section may be reinforced by flat-wire metal spirals. The exit connecting portion **5** may be made directly from the same piece of material as that of the inflow lumen and its walls. It may also be independently made of thin wall metal material such as stainless steel and nitinol. The exit connecting portion **5** may also be configured as a separate piece, which can then be attached to the exit opening of the inflow lumen. The attachment may be realized by any conventional mechanisms such as screw fit, snap fit, friction fit, or by suitable adhesives.

Similarly, the outflow lumen **2** as shown in FIG. 1 is substantially linear in its longitudinal axis and circular in shape for its cross-section. However, it will be understood that it may have any desirable cross-sectional shape and/or configuration as may be expeditious for particular surgical application or convenient for manufacturing purpose. The diameter of the outflow lumen **2** may be from about 0.5 mm to about 2.5 mm. Preferably, it may be from about 1.0 mm to about 2.5 mm. More preferably, it may be from about 1.0 mm to about 1.5 mm.

The outer wall **10** of the outflow lumen may be configured to have identical wall thickness along the whole longitudinal length of the inflow lumen. It may also be configured to have varying wall thicknesses along the longitudinal length. Similarly, the inner wall **12** of the outflow lumen may be configured to have identical wall thickness along the whole longitudinal length. It may also be configured to have varying wall thicknesses along the longitudinal length. The thickness of both walls may be in the range of about 0.1 mm to about 1.0 mm. Preferably, the thickness of both walls may be in the range of about 0.2 mm to about 0.5 mm. More preferably, the thickness of both walls may be in the range of about 0.2 mm to about 0.4 mm. In addition, the outer wall **10** and the inner wall **12** of the outflow lumen may have the same or different wall thickness, even though it may be preferred that both have substantially identical thickness.

The receiving opening **8** of the outflow lumen as shown in FIG. 1 has a smooth curved shape. However, it will be understood that it may have any desirable cross-sectional shape and/or configuration as may be expeditious for particular surgical application or convenient for manufacturing purpose. The receiving opening **8** of the outflow lumen as shown tapers from a substantially circular cross-section shape at the beginning of the opening to a semi-circle cross-section shape towards the end of the opening. The end of the opening may also be configured to be substantially smaller than a semi-circle cross-section shape. The tapering may also be in straight cut, wavy cut, smooth curved cut, or irregular smooth curved shape. The receiving opening **8** may be configured to have a length of about 1 mm to about 5 mm along the longitudinal axis (the distance "b" as shown in FIG. 1). All these shapes/configurations may be achieved through conventional methods/techniques such as laser related techniques.

The receiving connecting portion **6** may be configured to have similar cross-sectional shape or configuration as that of the exit opening or the receiving opening. However, it will be understood that it may have any desirable cross-sectional shape and/or configuration as may be expeditious for particular surgical application or convenient for manufacturing purpose. It may also be configured to have the substantially the same longitudinal length as that of the receiving opening **8**, which may be in the range of about 1 mm to about 5 mm. It can also have a different longitudinal length from that of the receiving opening **8.** The receiving connecting portion **6** may also be combined with the exit connecting portion **4** of the inflow lumen to form one piece for the convenience of manufacturing.

The pressure sensor **21** of the outflow lumen (outflow pressure sensor) is shown to be disposed at the tip of the outflow section. It may also be disposed at the proximal end of the outflow lumen (not shown). It serves to provide information to the processing unit and/or controller to control and/or monitor the outflow pressure. It may also be configured to serve to monitor the pressure of the kidney for the safety of the subject under operation. The outflow pressure sensor can be any of those described for inflow pressure sensor **20.** It can also be made and used in a very similarly fashion as that for the pressure sensor **20.**

The outflow lumen **11** and its walls **10** and **12** may be made from known polymeric materials such as silicone, polyurethane, and polytetrafluoroethylene as previously described The outflow lumen walls **10** and **12** can be strengthened by metal spirals for varying pressure needs. In some preferred aspects, the whole outflow section may be reinforced by flat-wire metal spirals. The receiving connecting portion **6** may be made directly from the same piece of material as that of the outflow lumen and its walls. It may also be independently made of thin wall metal material such as stainless steel and nitinol. The receiving connecting portion **6** may also be configured as a separate piece, which can then be attached to the exit opening of the outflow lumen. The attachment may be realized by any conventional mechanisms such as screw fit, snap fit, friction fit, or by suitable adhesives. Even though the exit connecting portion **5** and the receiving connecting portion **6** are separately described and illustrated in FIG.1, it will be understood that they can be configured to be made from the same piece of material with two ends: one end for its connection with the exit opening of the inflow lumen, the other end for its connection with the receiving opening of the outflow lumen. Moreover, the exit connecting portion **5** and the receiving connecting portion **6** may be made from the same piece of material as that for the inflow lumen and its walls and the outflow lumen and its walls.

Even though the medical device 100 of FIG. 1 shows that the inner wall **13** of the inflow lumen **1** and the inner wall **12** of the outflow lumen **11** are separate, it is to be understood that the two walls can be merged into just one wall. In some embodiments, this may be preferred for manufacturing purposes. In some aspects, the thickness of the merged wall may be configured to be substantially identical to that of the outer wall of the inflow lumen or to that of the outer wall of the outflow lumen. In some other aspects, the thickness of the merged wall may be configured to be different from that of the outer wall of the inflow lumen or from that of the outer wall of the outflow lumen due to different surgical requirements or needs. In yet some preferred aspects, the inflow lumen section and the outflow lumen section of the medical device may be made from the same piece of material through known methods/techniques such as laser techniques.

When a medical device 100 of FIG. 1 is put into operation such as using a ureteral access sheath, the irrigation liquid can flow inside the inflow lumen **1** in the direction as illustrated by arrows **2**, out from exit opening **4**, and into the outflow lumen **11** through the receiving opening **8** flowing out of the surgical field in the direction shown by arrows **9.** A lower pressure area in the open space **7** will be created due to the Bernoulli effect, thus sucking small stones such as those with diameter less than 1 mm into the open space area, and then into the outflow lumen through an external source of suction such as a vacuum pump, a suction pump, a pulsing suction pump or aspirator to provide the suction driving force required for evacuating the small stones from the surgical field. As previously described, both the pressure of the irrigation liquid inside the inflow lumen and the pressure of the liquid inside the outflow lumen can be monitored and/or controlled through the pressure sensors, the processing unit, and controllers.

The flow rate of the liquid flowing in the inflow lumen **1** can be controlled and/or monitored by a processing unit or a controller through a valve at the proximal end of the device (not shown in the drawing) or through other mechanisms known in the art. Similarly, the flow rate of the liquid flowing out of the outflow lumen **11** can be controlled and/or monitored by a processing unit or controller through a valve at the proximal end of the device (not shown in the drawing) or through other mechanisms known in the art.

During a surgical or medical operation, the pressure sensors begin to detect the pressures for their respective lumens or locations. These pressure readings can be taken constantly, periodically, or on demand. The pressure readings can be taken many times a second or minute, and/or continuously adjusted so that the pressure variations are minimized. The pressure readings are transmitted to the processing unit and/or controller. If the pressure exceeds the desired or pre-set value, the processing unit can then directs the adjustments either through the irrigation flow or the suction flow until the pressure reaches the desired pressure.

In addition, more than two pressure sensors may be needed if necessary. These additional pressure sensors may be placed at the tip of the device. They may also be placed at the proximal end of the device. Moreover, other types of sensors such as temperature sensors and flow rate sensors may also be placed if desired to monitor other parameters. These sensors may also be disposed at the tip of the device.

In certain aspects, the above-mentioned method can also include inputting a desired pressure value into an input control such as a processing unit or a central controller so that the desired pressure is maintained. In certain aspects, the processing unit can be configured to control fluid through at least one of the inflow lumen and the outflow lumen based on the pressure measurement. In certain aspects, the processing unit can be configured to increase the flow of an irrigating fluid through the inflow lumen when the pressure measurement is less than the desired pressure value. In certain aspects, the processor can be configured to increase the flow of a fluid out of the outflow lumen when the pressure measurement is greater than the desired pressure value.

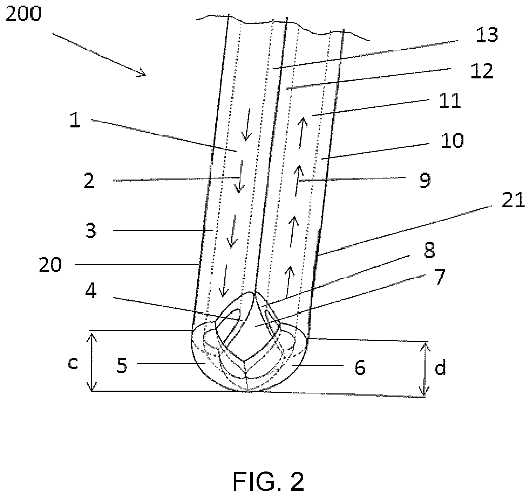

FIG. 2 illustrates a medical device 200 in accordance with one aspect of the present disclosure wherein the exit opening of the inflow lumen and the receiving opening of the outflow lumen of the medical device are substantially identical and straight cut. Substantially similar to what was described for FIG. 1, the difference is just that the device 200 has a straight cut exit opening **4** and a straight cut receiving opening **8.**

The exit opening **4** as shown in FIG. 2 has a straight cut shape. It may have a longitudinal length of about 1 mm to about 5 mm along the longitudinal axis. The receiving opening **8** as shown in FIG. 2 has a straight cut shape. It may also have a longitudinal length of about 1 mm to 5 mm along the longitudinal axis.

The connecting portion **5** of FIG. 2 may be configured to have similar cross-sectional shape or configuration as that of the exit opening or the receiving opening. However, it will be understood that it may have any desirable cross-sectional shape and/or configuration as may be expeditious for particular surgical application or convenient for manufacturing purpose. It may also have a longitudinal length as that of the exit opening or the receiving opening, which may be in the range of about 1 mm to 5 mm (distance "c" as shown). It can also have a different longitudinal length from that of the exit opening or the receiving opening. The connecting portion **5** may be combined with the connecting portion **6** to form a whole piece for the convenience of manufacturing.

The connecting portion **6** of FIG. 2 may be configured to have similar cross-sectional shape or configuration as that of the exit opening or the receiving opening. However, it will be understood that it may have any desirable cross-sectional shape and/or configuration as may be expeditious for particular surgical application or convenient for manufacturing purpose. It may also have a longitudinal length as that of the exit opening or the receiving opening, which may be in the range of about 1 mm to 5 mm (distance "d" as shown). It can also have a different longitudinal length from that of the exit opening or the receiving opening. The connecting portion **6** may be combined with the connecting portion **5** to form a whole piece for the convenience of manufacturing.

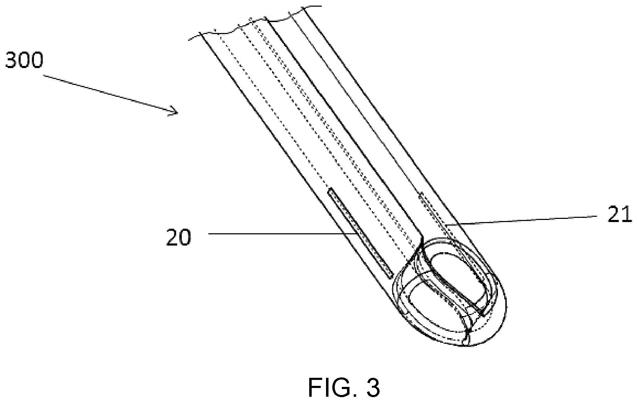

FIG. 3 illustrates a medical device 300 in accordance with one aspect of the present disclosure showing the disposition of both the inflow pressure sensor and the outflow pressure sensor. A medical device 300 as shown in FIG. 3 is identical to a medical device as depicted in FIG. 1 for a medical device 100. It is just a representation of a medical device 100 from a different angle for the purpose of showing the two pressure sensors. Consequently, all the descriptions for a medical device 100 are equally applicable to the medical device 300. The two pressure sensors as shown are symmetrically disposed on either side of the medical device. It is to be understood that the inflow pressure sensor may be disposed at any location on the side of the inflow lumen even at the proximal end of the inflow lumen (not shown). The same is true for the outflow lumen pressure sensor, meaning it may be disposed at any location on the side of the outflow lumen even at the proximal end of the outflow lumen (not shown). Moreover, it is contemplated that one pressure sensor may be sufficient for monitoring /controlling both the inflow lumen pressure and outflow lumen pressure, for example if disposed at the connecting portions of the device.

More specifically, the pressure sensors **20** and **21** may be made from an alloy of iron, titanium, copper and nickel having a dimension of about 10 mm in length, about 0.5 mm in width, and about 1.3 µm in thickness. Both pressure sensors may be disposed at a location which is about 6 mm to about 10 mm from the very tip of the medical device. In one preferred embodiment, the pressure sensors are disposed one either side of the device at a location of about 6.35 mm from the tip of the device.

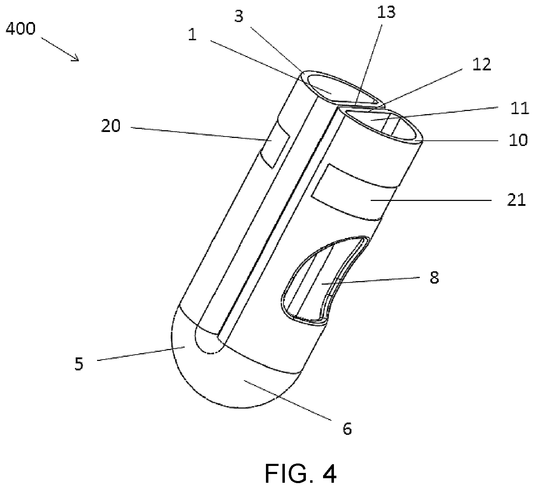

FIG. 4 is a cut-off and perspective illustration of a medical device 400 in accordance with one aspect of the present disclosure wherein the exit opening of the inflow lumen is merged into the outflow lumen with just one opening at the distal end of the outflow lumen.

The medical device 400 of FIG. 4 comprises an inflow section and an outflow section wherein the exit opening of the inflow lumen is completely merged into the outflow lumen. The inflow section as shown in FIG. 4 is the left side of the device without the exit opening. The inflow section includes an inflow lumen **1**, an outer wall **3**, an inner wall **13**, an exit opening connecting portion **5**, and a pressure sensor **20** disposed at the distal end of the inflow section. The outflow section includes a receiving opening connecting portion **6**, a receiving opening **8**, an outflow lumen **11,** an outer wall **10**, an inner wall **12**, and a pressure sensor **21** disposed at the distal end of the outflow lumen. It is to be noted that the connecting portions **5** and **6** are merged into one single piece as shown.

All those described for a device 100 of FIG. 1 may be similarly applicable to a device of 400 of FIG. 4 except one significant difference: the exit opening of the inflow lumen **1** is completely merged with the outflow lumen **11.** The only opening is the receiving opening **8** of the outflow lumen, which is used to let some of the irrigation liquid out. The opening **8** may have any desirable cross-sectional shape and/or configuration as may be expeditious for particular surgical application or convenient for manufacturing purpose. It may also have a longitudinal length in the range of about 1 mm to about 5 mm. It might also be worth pointing out that the cross-sectional shape of both the inflow lumen **1** and the outflow lumen **11** is substantially semi-circle, rather than the substantially circle cross-section as shown in FIG. 1 even though it may also be in any shape or configuration.

The two pressure sensors **20** and **21** as shown are symmetrically disposed on either side of the medical device. It is to be understood that the inflow pressure sensor may be disposed at any location on the side of the inflow lumen even at the proximal end of the inflow lumen (not shown). The same is true for the outflow lumen pressure sensor, meaning it may be disposed at any location on the side of the outflow lumen even at the proximal end of the outflow lumen (not shown). Moreover, it is contemplated that one pressure sensor may be sufficient for monitoring /controlling both the inflow lumen pressure and outflow lumen pressure, for example if disposed at the connecting portions (portions **5** and **6**) of the device.

It has to be understood that the materials and methods described for making a device 100 of FIG. 1 can be equally or similarly applicable for making a device 400 as described for FIG. 4.

While preferred embodiments of the present disclosure have been shown and described herein, it will be obvious to those skilled in the art that such embodiments are provided by way of example only. Numerous variations, changes, and substitutions will occur to those skilled in the art without departing from the disclosure. It should be understood that various alternatives to the embodiments of the disclosure described herein may be employed. It is intended that the following claims define the scope of the invention.

## Claims

1. A medical device, comprising:
an elongate member having a distal end and a proximal end;
an inflow lumen (1) disposed within the elongate member, the inflow lumen (1) having an outer wall (3), a proximal end, a distal end and a slanting exit opening (4) at the distal end of the inflow lumen (1);
an outflow lumen (11) disposed within the elongate member, the outflow lumen (11) having an outer wall (10), a proximal end, a distal end and a slanting receiving opening (8) at the distal end of the outflow lumen (11); and
a connecting portion (5, 6) connected to the exit opening (4) of the inflow lumen (1) and to the receiving opening (8) of the outflow lumen (11), the connecting portion (5, 6) surrounding an open space (7) at the distal end of the elongate member, where the exit opening (4) of the inflow lumen (1) meets the receiving opening (8) of the outflow lumen (11), such that liquid can flow out from exit opening (4), through the open space (7) at the distal end of the elongate member, and into the receiving opening (8).

2. The medical device of claim 1, wherein the device further comprises one pressure sensor (20, 21) disposed at the distal end of the elongate member.

3. The medical device of claim 1, wherein the device further comprises two pressure sensors (20, 21).

4. The medical device of claim 3, wherein one (21) of the two pressure sensors (20, 21) is disposed at the distal end of the inflow lumen (1), the other (21) at the distal end of the outflow lumen (11).

5. The medical device of any of claims 3 or 4, wherein both of the two pressure sensors (20, 21) are low pressure sensing sensors.

6. The medical device of any of claims 2 to 5, wherein the device further comprises a processing unit configured to control fluid flow through the inflow lumen (1) or through the outflow lumen (11) or both based on a pressure measurement.

7. A system for removing intracorporeal stones during a medical operation, the system comprising:
a medical device according to claim 1, wherein the elongate member is a sheath; two pressure sensors (20, 21) configured to generate pressure measurements when in operation; and
a processing unit configured to control fluid flow through the inflow lumen (1) or the outflow lumen (11) or both based on the pressure measurements.

8. The system of claim 7, wherein one (20) of the two pressure sensors (20, 21) is disposed at the distal end of the inflow lumen (1), and the other (21) at the distal end of the outflow lumen (11).

## Patentansprüche

1. Eine medizinische Vorrichtung, umfassend:
ein langgestrecktes Element mit einem distalen Ende und einem proximalen Ende;
ein Zuflusslumen (1), das innerhalb des langgestreckten Elements angeordnet ist, wobei das Zuflusslumen (1) eine Außenwand (3), ein proximales Ende, ein distales Ende und eine schräge Ausgangsöffnung (4) am distalen Ende des Zuflusslumens (1) aufweist;
ein Ausflusslumen (11), das in dem langgestreckten Element angeordnet ist, wobei das Ausflusslumen (11) eine Außenwand (10), ein proximales Ende, ein distales Ende und eine schräge Aufnahmeöffnung (8) an dem distalen Ende des Ausflusslumens (11) aufweist; und
einen Verbindungsabschnitt (5, 6), der mit der Ausgangsöffnung (4) des Zuflusslumens (1) und mit der Aufnahmeöffnung (8) des Ausflusslumens (11) verbunden ist, wobei der Verbindungsabschnitt (5, 6) einen offenen Raum (7) am distalen Ende des länglichen Elements umgibt, wo die Ausgangsöffnung (4) des Zuflusslumens (1) auf die Aufnahmeöffnung (8) des Ausflusslumens (11) trifft, so dass Flüssigkeit aus der Ausgangsöffnung (4) durch den offenen Raum (7) am distalen Ende des langgestreckten Elements und in die Aufnahmeöffnung (8) fließen kann.

2. Die medizinische Vorrichtung nach Anspruch 1, wobei die Vorrichtung weiterhin einen Drucksensor (20, 21) umfasst, der an dem distalen Ende des langgestreckten Elements angeordnet ist.

3. Die medizinische Vorrichtung nach Anspruch 1, wobei die Vorrichtung weiterhin zwei Drucksensoren (20, 21) umfasst.

4. Die medizinische Vorrichtung nach Anspruch 3, wobei einer (21) der zwei Drucksensoren (20, 21) am distalen Ende des Zuflusslumens (1) und der andere (21) am distalen Ende des Ausflusslumens (11) angeordnet ist.

5. Die medizinische Vorrichtung nach einem der Ansprüche 3 oder 4, wobei beide Drucksensoren (20, 21) Niederdrucksensoren sind.

6. Die medizinische Vorrichtung nach einem der Ansprüche 2 bis 5, wobei die Vorrichtung weiterhin eine Verarbeitungseinheit umfasst, die dazu eingerichtet ist, den Fluidstrom durch das Zuflusslumen (1) oder durch das Ausflusslumen (11) oder beides basierend auf einer Druckmessung zu steuern.

7. Ein System zur Entfernung von intrakorporalen Steinen während einer medizinischen Operation, wobei das System umfasst:
eine medizinische Vorrichtung nach Anspruch 1, wobei das langgestreckte Element eine Hülle ist; zwei Drucksensoren (20, 21), die dazu eingerichtet sind, während des Betriebs Druckmessungen zu erzeugen; und
eine Verarbeitungseinheit, die dazu eingerichtet ist, den Fluidstrom durch das Zuflusslumen (1) oder das Ausflusslumen (11) oder beide basierend auf den Druckmessungen zu steuern.

8. Das System nach Anspruch 7, wobei einer (20) der beiden Drucksensoren (20, 21) am distalen Ende des Zuflusslumens (1) und der andere (21) am distalen Ende des Ausflusslumens (11) angeordnet ist.

## Revendications

1. Un dispositif médical, comprenant :
un élément allongé ayant une extrémité distale et une extrémité proximale ;
une lumière d'entrée (1) disposée à l'intérieur de l'élément allongé, la lumière d'entrée (1) ayant une paroi extérieure (3), une extrémité proximale, une extrémité distale et une ouverture de sortie oblique (4) à l'extrémité distale de la lumière d'entrée (1) ;
une lumière de sortie (11) disposée à l'intérieur de l'élément allongé, la lumière de sortie (11) ayant une paroi extérieure (10), une extrémité proximale, une extrémité distale et une ouverture de réception oblique (8) à l'extrémité distale de la lumière de sortie (11) ; et
une partie de connexion (5, 6) reliée à l'ouverture de sortie (4) de la lumière d'entrée (1) et à l'ouverture de réception (8) de la lumière de sortie (11), la partie de connexion (5, 6) entourant un espace ouvert (7) à l'extrémité distale de l'élément allongé, où l'ouverture de sortie (4) de la lumière d'entrée (1) rencontre l'ouverture de réception (8) de la lumière de sortie (11), de sorte que le liquide peut s'écouler de l'ouverture de sortie (4), à travers l'espace ouvert (7) à l'extrémité distale de l'élément allongé, et dans l'ouverture de réception (8).

2. Le dispositif médical de la revendication 1, dans lequel le dispositif comprend en outre un capteur de pression (20, 21) disposé à l'extrémité distale de l'élément allongé.

3. Le dispositif médical de la revendication 1, dans lequel le dispositif comprend en outre deux capteurs de pression (20, 21).

4. Le dispositif médical de la revendication 3, dans lequel l'un (21) des deux capteurs de pression (20, 21) est disposé à l'extrémité distale de la lumière d'entrée (1), l'autre (21) à l'extrémité distale de la lumière de sortie (11).

5. Le dispositif médical de l'une des revendications 3 ou 4, dans lequel les deux capteurs de pression (20, 21) sont des capteurs de basse pression.

6. Le dispositif médical de l'une des revendications 2 à 5, dans lequel le dispositif comprend en outre une unité de contrôle configurée pour commander l'écoulement du fluide dans la lumière d'entrée (1) ou dans la lumière de sortie (11) ou dans les deux, en fonction des mesures de pression.

7. Un système pour enlever des pierres intracorporelles pendant une opération médicale, le système comprenant :
un dispositif médical selon la revendication 1, dans lequel l'élément allongé est une gaine ; deux capteurs de pression (20, 21) configurés pour générer des mesures de pression lorsqu'ils sont en fonctionnement ; et
une unité de contrôle configurée pour commander l'écoulement du fluide dans la lumière d'entrée (1) ou la lumière de sortie (11) ou les deux, en fonction des mesures de pression.

8. Le système de la revendication 7, dans lequel l'un (20) des deux capteurs de pression (20, 21) est disposé à l'extrémité distale de la lumière d'entrée (1), et l'autre (21) à l'extrémité distale de la lumière de sortie (11).
